# EUROPEAN PATENT SPECIFICATION

(11) **EP 4 429 725 B1**
(45) Date of publication and mention of the grant of the patent: **31.12.2025**
(21) Application number: 22802192.9
(22) Date of filing: 11.11.2022
(51) Int. Cl.: A61M 1/16, A61M 1/36

(54) **SELECTIVE PERFUSION DEVICE AND METHOD**
VORRICHTUNG UND VERFAHREN ZUR SELEKTIVEN PERFUSION
DISPOSITIF ET PROCÉDÉ DE PERFUSION SÉLECTIVE

(30) Priority: 12.11.2021 GB 202116394
(43) Date of publication of application: 18.09.2024
(73) Proprietor: VESTLANDETS INNOVASJONSSELSKAP AS (VIS), 5006 Bergen (NO)
(72) Inventor: TUSETH, Vegard, 5019 Bergen (NO); HALVORSEN LØLAND, Kjetil, 5053 Bergen (NO)
(74) Representative: Hoffmann Eitle
(86) International application number: PCT/EP2022/081622
(87) International publication number: WO 2023/084024

(56) References cited:
- WO-A1-2017/122377
- US-A1- 2004 162 519
- US-A1- 2018 272 104
- US-B1- 6 699 231
- US-B2- 9 108 029

## Description

The present invention relates to a life support device, and more particularly to a device for the selective perfusion of target areas of a patient's body so as to reduce the risk of irreversible damage and to improve the chances of survival.

### Background of the Invention

Without immediate medical intervention, the survival rate of patients suffering cardiac arrest is, on average, no more than 12%. Where the patient survives, the risk of permanent brain damage is high. In order to minimise these risks, it is crucial to maintain and/or restore the heart and brain function within moments of the cardiac event.

In a first instance, cardiopulmonary resuscitation (CPR) is performed to maintain blood flow, until heart function is restored. While CPR may improve the chances of survival, it only provides temporary and partial relief until the patient's heart is restored to a viable rhythm. Defibrillation is the most commonly used treatment to restore a normal heartbeat in emergencies, and immediate defibrillation increases the survival rate to over 70%.

CPR may provide limited circulatory support, a person in cardiac arrest does not breathe, so that the blood circulated via CPR is oxygen-depleted. Simultaneous respiratory support may be provided by giving rescue breaths to force air into the patient's lungs or, if available, using a respiratory support apparatus. The most commonly available respiratory support devices include endotracheal tubes and laryngeal masks, but the efficiency of these devices depends on the patient's airways and lungs being accessible and relatively undamaged, and also on there being sufficient circulation to perfuse the oxygenated blood.

In certain circumstances, the emergency services may have alternative devices for facilitating the delivery of oxygen to the patient's system. One such device is based on extracorporeal membrane oxygenation (ECMO). An ECMO device pumps blood outside the patient's body, and acts as an artificial lung by removing carbon dioxide and replacing it with oxygen. The oxygenated blood is pumped back into the patient, usually from/via the femoral vein to the femoral artery. When employed in a cardiac arrest treatment, this method is referred to as "E-CPR".

While an ECMO device relieves the lungs of their function, it does not provide the function of circulating blood around the patient, as would normally be effected by a healthy heart. In particular, the ECMO-oxygenated blood will be indiscriminately delivered (more or less efficiently by the cardiac support treatment) to all organs throughout the body. Furthermore, the tissues and organs of a cardiac arrest patient become engorged and saturated with fluids, so that the congested organs cannot efficiently absorb the oxygenated blood infused into the circulatory system.

Consequently, the risk of starving the brain of a cardiac arrest sufferer of oxygen remains.

Despite the prompt intervention of emergency services, the chances of survival remain low. As oxygen fails to reach critical organs, the likelihood of irreversible brain damage increases. There is therefore a need for critical care method and device for efficiently supplying oxygen (perfusion) to the patient's critical organs and preventing catastrophic and permanent damage.

It is an object of this invention to mitigate problems such as those described above and to provide an improved alternative to existing methods and devices.

Document US 2018/272104 A1 is considered as part of the prior art.

### Summary of Invention

The scope of the invention is limited to the set of claims.

According to a first aspect of the invention, there is
provided a system for the selective perfusion of one or more organs, said system comprising a first catheter comprising a means for occluding a first lumen downstream of said organ(s); a second catheter comprise a means for occluding a second lumen upstream of said organ(s); an extracorporeal device configured to deliver fluid through the first catheter and to receive fluid through the second catheter, and comprising means for processing the fluid, wherein the occlusion means act as a scaffold preventing the lumen from collapsing; and wherein the first catheter comprises a diffusor at the distal end of the catheter.

By way of illustration, there is described a method (which is excluded from the scope of the invention; this is also valid for the further occurrences of the term "method") for the selective treatment of one or more organs, said method comprising the step of compartmentalising said one or more organs by occluding a first lumen downstream of said organ(s)by means of a first catheter, and by occluding a second lumen upstream of said organ(s) by means of a second catheter; and the steps of extracting fluid from the compartment, processing the fluid and delivering the processed fluid to the compartment.

By way of illustration, there is described a system for the selective perfusion of one or more organs, said system comprising a first catheter comprising a means for occluding a first lumen downstream of said organ(s); a second catheter comprise a means for occluding a second lumen upstream of said organ(s); and means for actively draining fluid through the second catheter, and comprising means for processing the fluid. Within the context of the invention, "active drainage" may refer to the application of a drainage or suction force (for example using mechanical or manual means), as opposed to passive drainage (including gravitational drainage).

In the case of a critical organ oxygenation method, the brain and/or heart may be selectively oxygenated. In the event of cardiac arrest, blood circulation is halted, and the lungs are unable to oxygenate the patient's blood. The organs suffer hypoxia, oxygen-deprivation. Some organs and tissues may survive for hours or even days and/or be subsequently repaired. Brain activity ceases within 20 seconds; permanent brain damage starts after 3 to 4 minutes; and brain death occurs within 10 minutes from the cardiac event. The timescales for action on the heart are also precariously short. It is therefore critical to prioritise oxygenation of the brain and the heart.

CPR is an emergency procedure which can only provide temporary partial flow of blood, and the actual amount of oxygen reaching the brain from manual pumping is minimal. Airway intubation and methods, such as ECMO, fail or are unsuitable in that they provide oxygenation, but only partial or no circulation. Where some circulation is provided, the oxygenated blood is usually indiscriminately extracted from the vena cava and infused into the aorta from an access point in the femoral artery (in the groin area) and throughout the patient's circulatory system so that only a fraction of the circulated volume is extracted from and delivered to the brain and heart.

Furthermore, when the patient is in cardiac arrest, the blood vessels have a tendency to dilate so that the peripheral organs and tissues accommodate a relatively higher share of the circulated blood. Vital organs such as heart and brain can become congested with blood. In such a fluid-engorged state, the organs are unable to efficiently receive the oxygenated blood infused into the circulatory system.

The present invention provides a system for selectively perfusing (e.g. oxygenated blood to) one or more organs, in particular critical organs, such as the brain and/or heart. The present invention provides a system enabling the compartmentalisation a portion of the circulatory system and for providing selective treatment of the organs and/or within this compartment. The lumens are partially occluded so as to allow controlled circulation of (processed) fluid through the compartment. The first catheter occludes a lumen downstream of the compartment, and may include a channel in fluid communication with the compartment for delivering/infusing/feeding processed fluid. The second catheter occludes a lumen upstream of the compartment, and may include a channel in fluid communication with the compartment for draining fluid for subsequent processing.

The present invention enables the prioritised oxygenation of target/critical organs over the rest of the body, thereby minimising the risk of vital organ damage and death.

The present invention actively and effectively drains the congested critical organs of fluid. The fluid-drained organs can and will therefore imbibe the oxygenated blood infused into the circulatory system due to the fluid pressure gradient created by the enhanced drainage.

In methods, such as ECMO, the oxygenated blood is circulated throughout the patient's circulatory system. Consequently, a larger volume of blood is circulated. In a typical ECMO treatment, around 10 litres of fluid are circulated. By contrast, the method described herein only requires around 2 litres of fluid. This difference in volumes affects the fluid pressure required to suitably oxygenate the brain, and careful monitoring of the ECMO pressure and flow is essential to prevent injury and tissue damage. The lower volume used in the present invention means that the fluid may be infused less forcefully, at lower pressures.

A further issue encountered with ECMO devices is that it is not possible to apply active drainage force, because of the risk of the lumen (e.g. vein) collapsing onto itself under the suction force, thereby preventing effective blood drainage. If the drainage is ineffective, the compartment is saturated with blood and cannot be infused. The present invention is capable of actively draining the compartment by applying a drainage or suction pressure (e.g. using a pump), because the occlusion means acts as a supporting structure or scaffold preventing the lumen from collapsing. It is also possible to position the drainage catheter in areas of the circulatory system (such as anatomical chambers), which are less likely to collapse under suction.

Another advantage of the present invention is that smaller catheters may be used to circulate the fluid (for example, 3-5 mm diameter catheters in the present system vs. 6 to 10 mm diameter catheters for conventional ECMO catheters). When conventional ECMO is used in hospital settings, where imaging and other clinical techniques may be used to facilitate catheter insertion. However, in emergency situations, oxygenation support must be promptly established, with minimum risk and trauma on a cardiac arrest patient with no pulse, and hence little access visibility. The narrower catheter may be inserted through narrower portions of veins or arteries, and in the event of unsuccessful insertion, the catheter may be removed and re-introduced with minimum trauma.

As used herein, the term "means" can be equivalently expressed as, or substituted with, any of the following terms: device, apparatus, structure, part, sub-part, assembly, sub-assembly, machine, mechanism, article, medium, material, appliance, equipment, system, body or similar wording.

Within the context of the present invention, the terms "upstream" and "downstream" are defined relative to a native fluid flow. For example, "upstream of an organ" refers to the side of the organ from which fluid enters the organ in a native fluid flow, and "downstream of an organ" refers to the side of the organ from which fluid exits the organ in a native flow.

Within the context of the present invention, the terms proximal" and "distal" are defined relative to the access point of the catheter into the patient. For example, the "proximal end of a catheter" refers to the end of the catheter closest to the access point and the "distal end of a catheter" refers to the end of the catheter refers to the furthest from the access point.

### Accompanying figures

The invention will be further described with reference to the drawings and figures, in which
Figure 1 is a schematic representation of natural blood circulation in a human patient;
Figure 2 is a schematic representation of a conventional ECMO system;
Figure 3 is a schematic representation of a system according to the present invention;
Figure 4 is a schematic representation of a catheter used in a system according to the present invention;
Figures 5A to 5D are schematic representations of functional components of a system according to the present invention; and
Figures 6A to 6C are schematic representations of a puncture device used in a system according to the present invention.

The embodiments described herein are provided as exemplary and non-limiting embodiments of the present invention.

### Detailed description

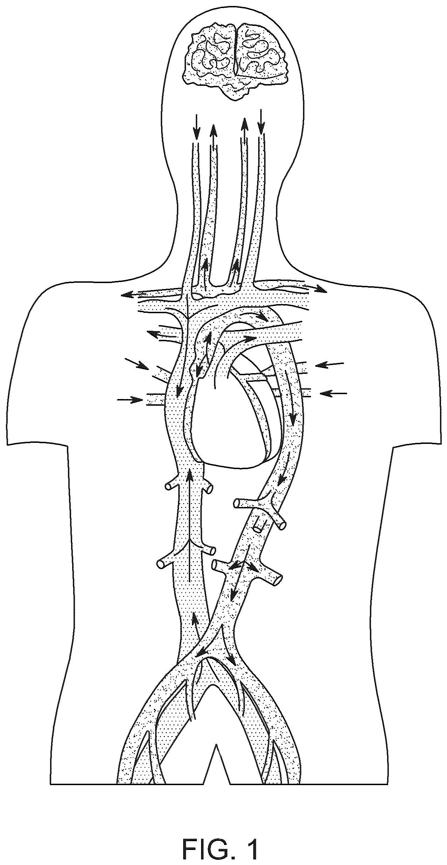

For reference purposes, Figure 1 illustrates the native blood circulation in a patient. The heart pumps blood through the circulatory system. Oxygen-depleted blood leaves the organs is pumped by and through the heart, and is oxygenated by the lungs. Oxygenated blood is recirculated to throughout the patient's circulatory system and redistributed to the organ.

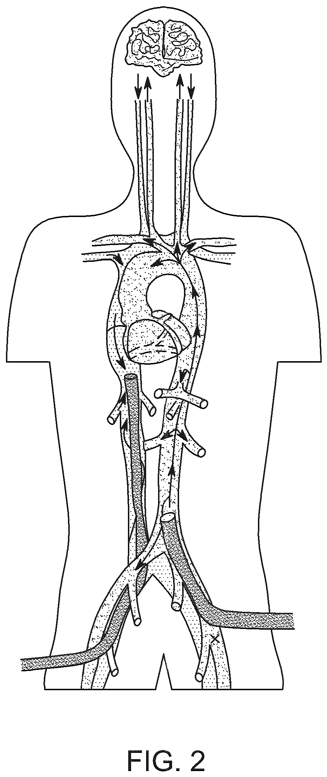

In the event of cardiac arrest, blood oxygenation is halted, and a device, such as an ECMO device, may be used as an artificial heart-lung machine. Figure 2 illustrates a conventional ECMO treatment, in which blood is extracted via the femoral vein (usually by gravitational force), oxygenated and infused back into the patient via the femoral artery. Cardiac arrest patients are susceptible to have an increased risk of vital organ congestion and edemas which may impair or completely stop blood flow to the organs. Increased pressure may be required to deliver blood past the existing congestion and edemas and improved drainage may be needed to reduce congestion/edemas. With a typical ECMO flow rate of between 4 to 6 litres per minute at the device, this pressure decreases by the time blood has reached the patient's brain and heart.

The oxygenated blood is distributed throughout the patient's whole circulatory system, so that the actual amount of oxygenated blood delivered to and drained from the brain and the heart is further compromised.

The access points will in part be dictated by accessibility to the circulatory system. It may be that the patient has sustained injuries or received treatment in the desired access area which makes insertion difficult. In practice, the ECMO flow is delivered in a retrograde manner, i.e. against the native blood flow, due to accessibility constraints.

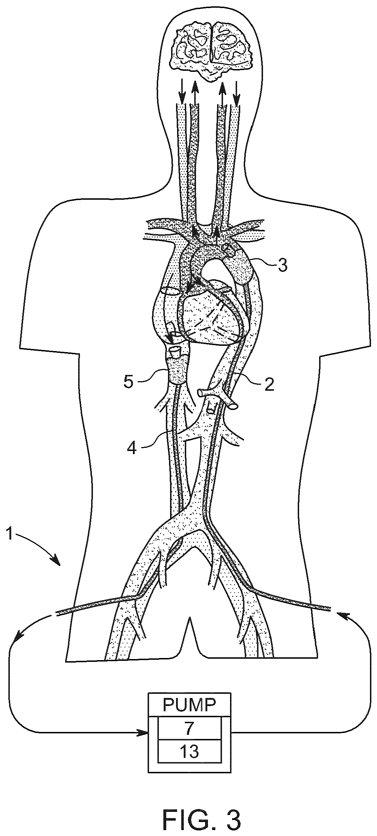

With reference to Figure 3, there is illustrated a system 1 for the selective treatment of one or more organs, said system 1 comprising a first catheter 2 comprising a means 3 for occluding a first lumen downstream of said organ(s); a second catheter 4 comprise a means 5 for occluding a second lumen upstream of said organ(s); an extracorporeal device 6 configured to deliver fluid through the first catheter 2 and to receive fluid through the second catheter 4, and comprising means 7 for processing the fluid.

In this embodiment, the access point of the first catheter 2 is the femoral artery, for example adjacent the patient's groin area. The access point of the second catheter 4 may be the femoral vein, for example also adjacent the patient's groin area. Other access points include, but are not limited to transfemoral, transaxillary/subclavian, transaortic, transapical, transcaval, transiliac and transcarotid access points. The access point of the first catheter 2 and the access point of the second catheter 4 may be adjacent, or may be remote. For example, the access point of the first catheter 2 may be in an artery, such as the femoral artery, and the access point of the second catheter 4 may be in a vein, such as the jugular vein (i.e. closer to the brain). In some embodiments, the first catheter 2 may be in arterial femoral artery or the subclavian artery. In some embodiments, the second catheter 4 may be in the jugular vein.

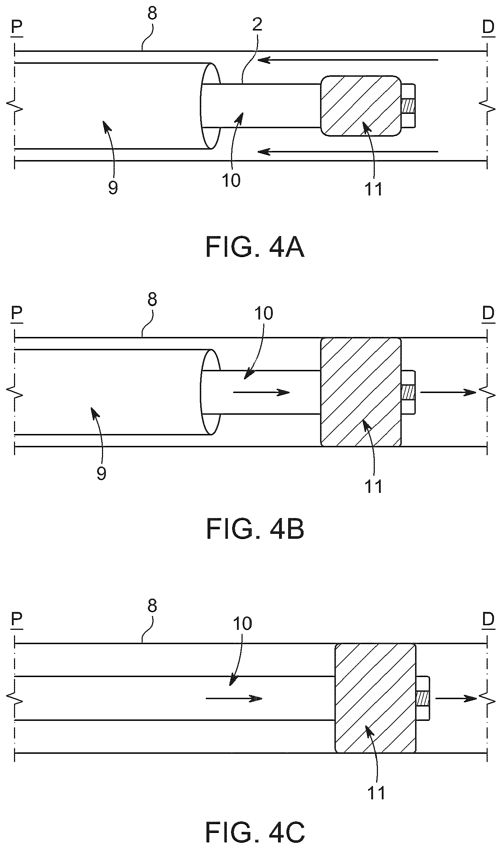

With reference to figures 4A to 4C, the proximal side (closer the access point of the catheter) is designated by P, and the distal side (further from the access point of the catheter) is designated by D. The first and second catheter 2, 3 may be configured in the same manner, or may have different structures. The catheters 2,4 will be described using a first catheter 2 as an example. The features described with reference to the first catheter 2 are applicable to the second catheter 4, unless otherwise specified.

The first catheter 2 may be inserted through an access point (not shown) and advanced through a lumen 8 (e.g. femoral artery) of the patient by means of a delivery sheath 9.

The catheter 2 comprises a channel 10 for fluid communication with an extracorporeal device 6. In this embodiment, the first catheter 2 comprises a channel 10 for delivering processed (e.g. oxygenated) blood into the patient's, and the second catheter 4 comprises a channel for draining (e.g. oxygen-depleted) blood from the patient for subsequent delivery to the extracorporeal device 6.

The catheter 2 comprises means 3 for occluding a lumen. Preferably, the occlusion means comprises an expandable structure 11.

The expandable structure 11 may be coupled to or adjacent the distal end of the catheter 2, so as not to hinder the distal opening of the catheter 2. Preferably, distal end of the catheter 2 does not substantially extend beyond the expandable structure 11, and in some embodiment, the distal opening of the catheter 2 is flush with the outer surface of the expanded occlusion means 3.

The expandable structure 11 may be folded in a delivery configuration (see figure 4A) so as to fit within the delivery sheath 9, and may be expanded into an occluding configuration (see figure 4B and 4C). In the occluding configuration, the occluding means 3 may occlude the interstitial space between the outer surface of the catheter 2 and the inner surface of the lumen 8. The expandable structure 11 may comprise an expandable scaffold and/or an inflatable balloon, which may be controlled extracorporeally by the medical practitioner.

In the occluding configuration, oxygenated blood may circulate from the extracorporeal device 6 through the channel 10 of the catheter and be delivered exclusively to the target compartment (i.e. between the occlusion means of the first catheter 2 and the occlusion means of the second catheter 4).

The catheter 2 may comprise a one or more fluid pressure sensors 12. The one of more fluid pressure sensors 12 may be located at or beyond the distal end of the catheter 2. The sensor (s) 12 may be coupled to the catheter 2, or may be separately provided through the channel 10. The pressure sensor(s) preferably measures the pressure of the fluid being delivered (or drained) at the delivery (or drainage) point, i.e. adjacent the distal end of the catheter 2 (or catheter 4). The detected pressures may be monitored in order to adjust the delivery and/or drainage pressure.

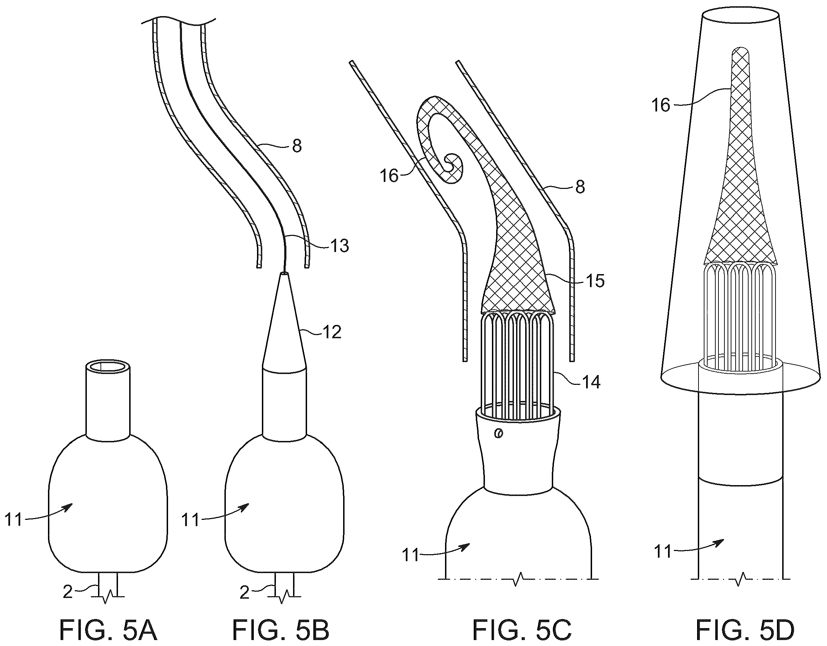

With reference to Figures 5A to 5D, the catheter 2 may comprise one or more additional functional components, preferably at or adjacent the distal end of the catheter 2.

Figure 5A illustrates a catheter 2 with an occlusion balloon 11. The distal end of the catheter extends from the occlusion balloon 11. Figure 5B illustrate a catheter 2 comprising a dilator 12, with a guide wire 13 slidably extending therethrough.

Figures 5C and 5D illustrate a diffusor 14 located at the distal end of the catheter 2. The diffusor 14 may comprise a plurality of apertures to allow the fluid to flow freely therethrough and preferably to direct the flow of fluid. This is advantageous in that it mitigates the effects of retrograde infusion of fluid against the native blood flow.

A puncture component 15 is coupled to the distal end of the diffusor 14, but may alternatively be couple to the distal end of the catheter 2. The puncture component 15 comprises a flexible tip 16. In Figure 5D, the flexible tip 16 is in a working/puncture configuration, and may be maintained in said puncture configuration by means of a cover 17. The cover 17 may be conical or tapered, so as to serve as a dilator. In Figure 5C, the flexible tip 16 is in a non-functional configuration. For example, the flexible tip 16 is curled upon itself so as to shield the sharp puncturing end thereof. In this configuration, the flexible tip 16 may be advanced atraumatically through the lumen, without the risk of accidental puncture. The puncture component 15 may be conical or tapered to facilitate puncture and insertion through the lumen wall. The puncture component 15 may be arranged and configured to allow fluid flow therethrough. For example, the puncture component 15 may comprise or consist of a mesh.

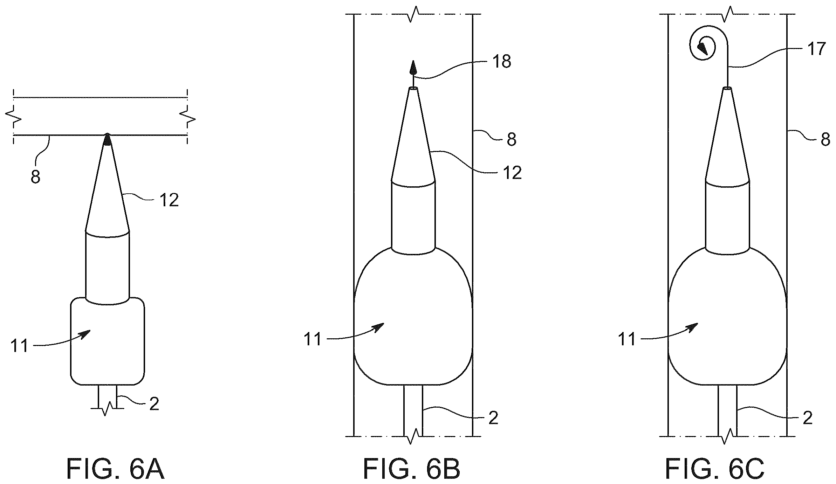

An alternative puncture component 17 is illustrated in Figure 6A to 6C. In this embodiment, the catheter 2 comprises an occlusion means 11, preferably a dilator, and a puncture component 17 with a puncture tip 18. The puncture component 17 slidably extends through the dilator 12. The dilator 12 comprises a distal opening. The inner dimensions of the distal opening of the dilator 12 may be such that the puncture tip 18 is prevented from sliding back into the dilator 12 or the catheter 2. Figure 6A illustrates the puncture component 17 in a puncture configuration. In Figure 6B, the lumen wall has been punctured and the dilator 12 dilates the puncture for access. In Figure 6C, the flexible distal portion of the puncture component 17 curls upon itself to shield the puncture tip 18.

The flexible tip 16 and the puncture component 17 may comprise or consist of a shape-memory material, such as nitinol, so that the flexible tip 16 and the puncture component 17 have a set atraumatic (for example curled) configuration, and a stretched configuration for puncture.

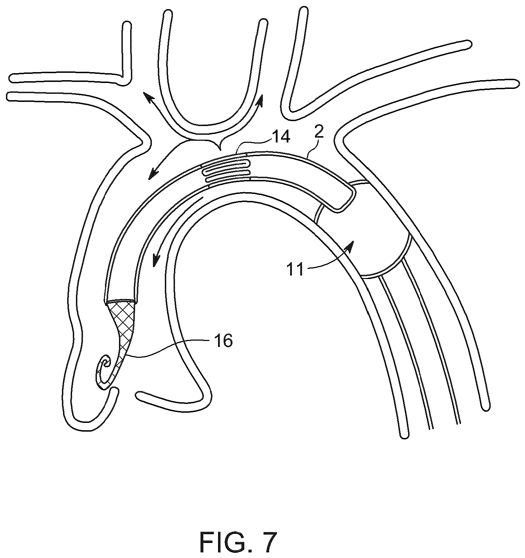

With reference to Figure 7, the one or more functional components may be located at any location adjacent the distal end of the catheter, provided they are located distally from the occlusion means 11. The catheters 2,4 may comprise one or more diffusors 14 and/or one or more occlusion means 11. The functional components can preferably be controlled and manipulated separately by the medical practitioner, who is then able to selectively and individually treat different parts of the patient's anatomy. For example, the patient may have been injured and suffer from haemorrhage. In this situation, it is possible to compartmentalise the patient's circulatory system (by strategically positioning occlusion means) so as to distribute less or no blood and/or blood thinners to the injured area.

The present invention also enables the administration of a first treatment of a first target area and a second treatment of a second target area, for example by segmenting the catheter 2 using multiple occlusion means 11. The channel 10 may comprise a multi-lumen structure to support the different treatments.

The system may comprise means 13 for controlling the fluid flow to and/or from the extracorporeal device 6. Pressure control may be effected by means of an integrated flow optimisation algorithm. The extracorporeal device 6 may comprise a pump, such as a centrifugal pump (not shown).

The extracorporeal device 6 comprises means 7 for processing a fluid. In a preferred embodiment, the processing means 7 comprise means for oxygenating the fluid, an oxygenator, such as a membrane oxygenator. Oxygen-depleted blood is drained from the patient, in particular from the target compartment, via the second catheter 4, oxygenated through the extracorporeal device 6, and oxygenated blood is delivered to the compartment via the first catheter 2.

The processing means 7 may alternatively or additionally comprise means for cooling and/or heating. This feature may be beneficial in the case of hypothermic treatment. The temperature of the fluid may be lowered in order to lower the body temperature. This prevent or reduces brain damage during cardiac arrest. Alternatively, the fluid may be heated in the context of a thermal treatment sometimes used in cancer patient. A tumour may be compartmentalised using the present system, and selectively heated to kill cancerous cells.

The processing means 7 may alternatively or additionally comprise means for delivering one or more compounds to the fluid. It may for example be beneficial to administer one or more of a blood thinning compound, a thrombolytic compound, an anti-inflammatory compound, an anti-swelling compound, and the like. In the case of a cancer treatment, it may be beneficial to administer one or more cytotoxic drugs, with or without heating the fluid.

The processing means 7 may alternatively or additionally comprise means for removing one or more compounds from the fluid. For example, the processing means 7 may remove carbon dioxide, toxins and/or drugs from the fluid.

The present system may be used in a method for the selective treatment of one or more organs, said method comprising the step of compartmentalising said one or more organs by occluding a first lumen downstream of said organ(s)by means of a first catheter, and by occluding a second lumen upstream of said organ(s) by means of a second catheter; and the steps of extracting fluid from the compartment, processing the fluid and delivering the processed fluid to the compartment.

The method enables the compartmentalisation and selective treatment and/or perfusion of critical organs, by occluding upstream and upstream of the target organs. Blood circulates through the extracorporeal device 6, for example for oxygenation and carbon dioxide removal. The oxygenated blood flows through the first catheter 2, by-passing non-critical organs, and is delivered to the target compartment to be treated. Oxygen is delivered to the critical organs (such as the brain, the heart and lungs), and oxygen-depleted blood is drained through the second catheter 4. Therefore, the bulk of the oxygen reaches the critical organs, which are prioritised over non-critical organs. There is negligible loss of pressure as the oxygenated blood is delivered directly to the compartment.

In the event of cardiac arrest, the first responder will typically apply Cardio-Pulmonary Resuscitation (CPR), in order to maintain partial blood flow to the brain and preserve brain function. CPR relieves the heart from its pumping function, and oxygen is mechanically circulated to the patient's blood for oxygenation, until the patient is connected to the present system.

The medical responder incises the patient's skin in the groin area (or other area close to a suitable lumen) to access the femoral artery. A delivery sheath 8 is inserted through the access point into the artery. The system 1 may comprise one or more functional components in order to facilitate the puncture of the lumen wall (e.g. a puncture needle, a flexible tip 16 or a puncture component 17) and to facilitate the insertion of the system 1 into the lumen (e.g. a dilator 12).

Should the sheath 8 be improperly inserted, the step is repeated, atraumatically owing to the small catheter diameter (preferably from 3 to 5 mm diameter). The sheath 8 is advanced through the patient's circulatory system until its distal end is adjacent the intended occlusion site downstream of the critical organ(s). The first catheter 2 is inserted into and advanced through the sheath 8, with the expandable structure 11 in a folded, delivery configuration. The first catheter 2 is inserted so as to infuse the lumen in a retrograde manner against the native flow. The first catheter 2 is advanced beyond the distal opening of the sheath 8, and the expandable structure 11 is deployed into an occluding/working configuration. In the deployed configuration, the occluding means 3 prevents fluid circulation into the compartment, except through the channel 10 of the first catheter 2.

A delivery sheath 8 is inserted through a second access point to the femoral vein (or any other suitable lumen). The second catheter 4 is inserted so as to drain the lumen countercurrent to the native flow. The sheath 8 is advanced through the patient's circulatory system until its distal end is adjacent the intended occlusion site upstream of the critical organ(s). The second catheter 4 is inserted into and advanced through the sheath 8, with the expandable structure 11 in a folded, delivery configuration. The second catheter 4 is advanced beyond the distal opening of the sheath 8, and the expandable structure 11 is deployed into an occluding/working configuration. In the deployed configuration, the occluding means 3 prevents fluid circulation from the compartment, except through the channel 10 of the second catheter 4.

Blood in the circulatory system outside the compartment is stagnant, and the system 1 does not deliver fluid to the organs and tissues outside compartment. Thus, the present system is most beneficial is emergency and critical situations.

In the example of Figure 3, the most critical organs are the brain and the heart, but the compartment may also include the lungs. Therefore, a fluid circulation circuit is formed comprising the extracorporeal device 1, the first catheter 2, the heart, the lungs, the brain and the second catheter 4.

The first catheter 2 may occlude the aorta, adjacent but beyond the bifurcation to the cerebral vessels.

The second catheter 4 may occlude the inferior vena cava, preferably adjacent the heart.

It is envisaged that the method may be used for the treatment of other organs and/or tissues, with suitable upstream and downstream occlusion sites. Other organs and/or tissues may be treated, provided a suitable compartment can be isolated by occluding lumens at strategic sites. The choice of the occlusion sites may depend on the accessibility of the area to be treated. Certain parts of the patient may be inaccessible, if the patient is trapped after an accident or has been injured. It may also be that relevant access areas are not accessible due to prior treatments or procedures on the patient.

The first catheter 2 and the second catheter 4 are in fluid communication with the extracorporeal device 6. The first catheter 1 may be fluidly coupled to a fluid outlet of the extracorporeal device 6 and the second catheter 4 may be fluidly coupled to an inlet for receiving fluid from the second catheter 4.

The extracorporeal device 6 drains blood from the compartment via the second catheter 4, for example by means of a pump, such as a centrifugal pump. A key aspect of the present invention is that the system 1 drains blood from the compartment, thereby effectively drawing fluids from the crucial organs. When the patient is in cardiac arrest, the blood vessels have a tendency to dilate so that the tissues become congested with blood and drainage is impaired. The active and selective drainage achieved by the present system creates a fluid pressure differential in the critical organs, which forces the take-up of oxygenated blood by the fluid-drained critical organs by enhanced perfusion pressure. The enhanced drainage also allows the removal of toxins and other undesirable compounds from the congested organs.

The oxygen-depleted blood drained from compartment is processed by the extracorporeal device 6. The process step may include any one or more steps selected from adding one or more compounds (e.g. oxygen and/or one or more drugs) to the fluid, removing one or more compounds (e.g. carbon dioxide and/or toxins), heating and/or cooling the fluid.

The fluid pressure may be monitored at one or more locations. Preferably, the fluid pressure is measured as the fluid exits the first catheter 2 and/or as the fluid enters the second catheter 4. The pressure may be measured by means of one or more sensors coupled to the catheter(s) or used in conjunction with the catheter(s). The pressure readings may be entered in a control processor to adjust the incoming and outgoing fluid pressure.

The fluid flow is controlled and adjusted based on parameters such as the processing (e.g. oxygenation) rate of the extracorporeal device 6, the blood pressure of the patient, the pressure differential between the fluid delivery pressure and the fluid extraction pressure. In conventional ECMO devices, a balance must be struck between the required physiological blood flow and the capacity of the device to process large volumes of fluid. Since the method requires a smaller volume of fluid to be circulated, it is possible to achieve an efficient fluid processing, whilst maintaining adequate pressures.

The catheters 2,4 may be configured and arranged to partially or completely occlude the lumen 8, so that the inflow and/or outflow of oxygenated blood may be adjusted. The degree of occlusion (e.g., the degree of expansion of the expandable structure) may be adjusted to fit the intended purpose Alternatively or additionally, the occlusion means may be intermittently opened/closed. For example, it may be required to create a restricted, fully closed compartment around the critical organ(s), or to allow some oxygenated blood to be delivered to other organs. The diverted portion of the oxygenated blood may be homogeneously delivered to the rest of the patient's body, or may be directed to specific organs (other than the primary critical organs). The latter may for example be achieved by dividing the patient's circulatory system into several compartments by strategically positioning a plurality of catheters. The degree of irrigation of each such compartment may be individually adjusted.

In the simplest embodiment, two catheters 2,4 are positioned to create a first compartment around one or more critical organs (e.g., the brain and/or heart). Processed fluid (e.g., oxygenated blood) is circulated throughout this first compartment using a first processing means. The rest of the circulatory system outside the first compartment, i.e., the second compartment, may be treated separately, by circulating fluid processed by a second processing means, set under the same or different conditions as the first processing means.

This compartmentalisation may also be beneficial where the patient is bleeding. The present system may be used to prevent or minimise blood loss, by positioning the catheters to isolate the breach area.

Although the present invention has been described within the context of the critical oxygenation of the brain and heart, it is envisaged that it could have other advantageous implementations involving the selective treatment of organs and/or tissues within a compartment. For example, treatments such as drug delivery are described herein which may find a beneficial application in other medical treatments such as localised chemotherapy, hypothermic and thermal treatments.

Thus, the present invention provides a system for the selective treatment of a patient's organs and/or tissues. The present invention allows an effective treatment of the target organs, by providing an improved and enhanced drainage of the target organs, which during cardiac arrest are congested with fluids and cannot be effectively infused.

The invention provides mitigates the problems encountered by conventional life support apparatus, and in particular by conventional ECMO devices. Critical organs can be prioritised so as to prevent or reduce the risk of brain damage, and ultimately so as to increase the survival rate of cardiac arrest patients.

## Claims

1. A system (1) for the selective perfusion of one or more organs, said system comprising
a first catheter (2) comprising a means (3) for occluding a first lumen downstream of said organ(s);
a second catheter (4) comprise a means (5) for occluding a second lumen upstream of said organ(s);
an extracorporeal device (6) configured to deliver fluid through the first catheter (2) and to receive fluid through the second catheter (4), and comprising means (7) for processing the fluid;
wherein the occlusion means (3) act as a scaffold preventing the lumen from collapsing; and
wherein the first catheter (2) comprises a diffusor (14) at the distal end of the catheter (2).

2. The system according to claim 1, wherein the occlusion means (3) comprises an expandable structure (11).

3. The system according to claim 2, wherein the expandable structure (11) comprises an expandable scaffold and/or an inflatable balloon.

4. The system according to any preceding claim, further comprises means for controlling the fluid flow to and/or from the extracorporeal device.

5. The system according to any preceding claim, wherein the system comprises one or more fluid pressure sensors (12), preferably at or beyond the distal end of the catheter.

6. The system according to any preceding claim, wherein the processing means comprises means for oxygenating the fluid.

7. The system according to any preceding claim, wherein the processing means comprises means for cooling and/or heating.

8. The system according to any preceding claim, wherein the processing means comprises means for delivering and/or removing one or more compounds to the fluid.

9. The system according to any preceding claim, wherein the organs include the brain and/or the heart.

## Patentansprüche

1. System (1) zur selektiven Perfusion von einem oder mehreren Organen, wobei das System Folgendes umfasst
einen ersten Katheter (2), der ein Mittel (3) zum Okkludieren eines ersten Lumens stromabwärts von dem/den Organ/en umfasst;
einen zweiten Katheter (4), der ein Mittel (5) zum Okkludieren eines zweiten Lumens stromaufwärts von dem/den Organ/en umfasst;
eine extrakorporale Vorrichtung (6), die so konfiguriert ist, dass sie Fluid durch den ersten Katheter (2) abgibt und Fluid durch den zweiten Katheter (4) empfängt und Mittel (7) zum Verarbeiten des Fluids umfasst;
wobei das Okklusionsmittel (3) als Gerüst wirkt, das Kollabieren des Lumens verhindert; und
wobei der erste Katheter (2) am distalen Ende des Katheters (2) einen Diffusor (14) umfasst.

2. System nach Anspruch 1, wobei das Okklusionsmittel (3) eine ausdehnbare Struktur (11) umfasst.

3. System nach Anspruch 2, wobei die ausdehnbare Struktur (11) ein ausdehnbares Gerüst und/oder einen aufblasbaren Ballon umfasst.

4. System nach einem vorhergehenden Anspruch, weiter umfassend Mittel zum Steuern des Fluidstroms zu und/oder von der extrakorporalen Vorrichtung.

5. System nach einem vorhergehenden Anspruch, wobei das System einen oder mehrere Fluiddrucksensoren (12), vorzugsweise an oder hinter dem distalen Ende des Katheters, umfasst.

6. System nach einem vorhergehenden Anspruch, wobei das Verarbeitungsmittel Mittel zur Oxygenieren des Fluids umfasst.

7. System nach einem vorhergehenden Anspruch, wobei das Verarbeitungsmittel Mittel zum Kühlen und/oder Erwärmen umfasst.

8. System nach einem vorhergehenden Anspruch, wobei das Verarbeitungsmittel Mittel zum Abgeben und/oder Entfernen von einer oder mehreren Verbindungen in bzw. aus dem Fluid umfasst.

9. System nach einem vorhergehenden Anspruch, wobei die Organe das Gehirn und/oder das Herz einschließt.

## Revendications

1. Système (1) de perfusion sélective d'un ou plusieurs organes, ledit système comprenant
un premier cathéter (2) comprenant un moyen (3) d'occlusion d'une première lumière en aval dudit ou desdits organes ;
un second cathéter (4) comprend un moyen (5) d'occlusion d'une seconde lumière en amont dudit ou desdits organes ;
un dispositif (6) extracorporel configuré pour délivrer du fluide à travers le premier cathéter (2) et pour recevoir du fluide à travers le second cathéter (4), et comprenant des moyens (7) de traitement du fluide ;
dans lequel les moyens (3) d'occlusion agissent comme un support empêchant la lumière de s'affaisser ; et
dans lequel le premier cathéter (2) comprend un diffuseur (14) à l'extrémité distale du cathéter (2).

2. Système selon la revendication 1, dans lequel le moyen (3) d'occlusion comprend une structure (11) expansible.

3. Système selon la revendication 2, dans lequel la structure (11) expansible comprend un support expansible et/ou un ballonnet gonflable.

4. Système selon une quelconque revendication précédente, comprenant en outre des moyens de contrôle du débit de fluide vers et/ou en provenance du dispositif extracorporel.

5. Système selon une quelconque revendication précédente, dans lequel le système comprend un ou plusieurs capteurs (12) de pression de fluide, de préférence au niveau ou au-delà de l'extrémité distale du cathéter.

6. Système selon une quelconque revendication précédente, dans lequel le moyen de traitement comprend des moyens d'oxygénation du fluide.

7. Système selon une quelconque revendication précédente, dans lequel le moyen de traitement comprend des moyens de refroidissement et/ou de chauffage.

8. Système selon une quelconque revendication précédente, dans lequel les moyens de traitement comprennent des moyens de délivrance et/ou d'élimination d'un ou plusieurs composés dans le fluide.

9. Système selon une quelconque revendication précédente, dans lequel les organes incluent le cerveau et/ou le cœur.
